# EUROPEAN PATENT SPECIFICATION

(11) **EP 3 833 200 B1**
(45) Date of publication and mention of the grant of the patent: **04.12.2024**
(21) Application number: 20859672.6
(22) Date of filing: 13.10.2020
(51) Int. Cl.: A24F 40/40, A61M 11/04, A61M 15/06, A24F 40/20, A24F 40/465

(54) **AEROSOL GENERATING DEVICE INCLUDING EJECTOR**
AEROSOLERZEUGUNGSVORRICHTUNG MIT EJEKTOR
DISPOSITIF DE GÉNÉRATION D'AÉROSOL COMPRENANT UN ÉJECTEUR

(30) Priority: 16.10.2019 KR 20190128722
(43) Date of publication of application: 16.06.2021
(73) Proprietor: KT&G Corporation, Daedeok-gu Daejeon 34337 (KR)
(72) Inventor: AN, Hwi Kyeong, Seoul 02801 (KR)
(74) Representative: Ter Meer Steinmeister & Partner
(86) International application number: PCT/KR2020/013923
(87) International publication number: WO 2021/075819

(56) References cited:
- WO-A1-2015/198015
- WO-A1-2016/124550
- WO-A1-2017/068096
- WO-A1-2017/072148
- WO-A1-2019/030167
- WO-A1-2019/030167
- WO-A2-2013/076098
- KR-A- 20190 029 780

## Description

### Technical Field

Embodiments relate to an aerosol generating device including an ejector for ejecting an aerosol generating article, and more particularly, to an aerosol generating device including an ejector having at least one hole portion on an outer circumferential surface.

### Background Art

Recently, the demand for alternative methods to overcome the shortcomings of general cigarettes has increased.

For example, there is growing demand for an aerosol generating device that generates the aerosol by heating an aerosol generating material such as cigarettes or cartridges, rather than by combusting cigarettes.

Accordingly, studies on a heating-type cigarette and a heating-type cartridge have been actively conducted.
WO 2013/076098 A2 relates to an extractor for an aerosol-generating device. The device is configured to receive a smoking article including an aerosol-forming substrate and comprises a heater for heating the aerosol-forming substrate to form the aerosol. The extractor is for extracting a smoking article received in the aerosol-generating device. The extractor comprises a sliding receptacle for receiving the smoking article, and a sleeve for receiving the sliding receptacle. The sliding receptacle is slidable in the sleeve between a first position in which the aerosol-forming substrate of the smoking article is positioned so as to be heated by the heater, and a second position in which the aerosol-forming substrate is substantially separated from the heater. The sliding receptacle includes a support to support the aerosol-forming substrate of the smoking article as the sliding receptacle and the smoking article are being moved from the first position to the second position.
WO 2016/124550 A1 relates to an elongated aerosol-generating device that is capable of receiving an aerosol-generating article and comprises a heater for heating the aerosol-generating article and an extractor for extracting an aerosol-forming article received in the aerosol-generating device. The heater extends longitudinally with respect to the elongated aerosol-generating device and is configured for penetrating an internal portion of the aerosol-generating article. The extractor is movably coupled to the aerosol-generating device between a first position and a second position, the first position being an operating position defined by the heater being in contact with the aerosol-generating article, and the second position being an extraction position defined by the heater being separated from aerosol-generating article.
WO 2019/030167 A1 relates to an aerosol-generating device comprising a housing having a chamber. An induction coil is disposed around at least a portion of the chamber. The device comprises a heating compartment for receiving an aerosol-generating article. The heating compartment is detachably insertable into the chamber of the housing. The heating compartment comprises a cavity configured to receive at least a portion of the aerosol-generating article. The device further comprises a heating element which is arrangeable within the cavity of the heating compartment. The heating element extends into the cavity in a longitudinal direction of the cavity. The heating element is configured to penetrate an aerosol-forming substrate comprised in the aerosol-generating article and received in the cavity.

### Disclosure of Invention

### Solution to Problem

Aerosol generating devices may include an ejector for easily ejecting an aerosol generating article accommodated in the aerosol generating device. An aerosol generating article may be inserted into the ejector, and the ejector may be detachably coupled to an accommodating portion for accommodating the aerosol generating article.

However, conventional ejector may wrap the outer surface of the aerosol generating article, so that the efficiency of heat transfer to the aerosol generating article may be reduced. Residues generated after the use of the aerosol generating article may be deposited in the ejector. When the efficiency of heat transfer is reduced and the deposition of residues is repeated, the flavor of the aerosol inhaled by a user may be reduced. In addition, residues in the aerosol generating device may adversely affect the user in terms of hygiene and health.

Embodiments provide an aerosol generating device including an ejector having at least one hole portion.

Embodiments provide an aerosol generating device including an ejector having a through hole which includes an expanding portion.

The technical problems to be solved by the present embodiments are not limited to the technical problems as described above, and other technical problems may be derived from the following embodiments.

### Advantageous Effects of Invention

In order to solve said problems, the present invention provides an aerosol generating device as defined in the present claim 1.

An aerosol generating device according to the present invention includes an ejector having at least one hole portion. Through the at least one hole portion of the ejector, the inside and outside of the ejector may communicate with each other. Heat generated outside the ejector may be efficiently transferred into the ejector through the at least one hole portion. Efficient heat transfer into the ejector may enhance the flavor of the generated aerosol, increase the amount of atomization, and eliminate heating imbalance of the aerosol generating article.

Generally, in the ejector of the aerosol generating device, foreign substances such as residues of the cut tobacco of the aerosol generating article and dust may be accumulated. Deposition of the residues may reduce the thermal efficiency of the aerosol generating device and reduce the flavor of the generated aerosol.

In the aerosol generating device according to the present embodiments, it may be easy to access to the inside of the ejector through the at least one hole portion. Through the access to the inside of the ejector, the user can easily remove the residues deposited in the ejector.

The ejector according to the present invention includes a through hole having an expanding portion which has a diameter increasing along a longitudinal direction of the ejector. Air passing through the through hole along an inclined wall may expand inside the ejector as it flow along the inclined wall. Accordingly, pressure loss in the ejector and the aerosol generating article may be prevented, and an airflow path is expanded to increase the inflow of air.

The airflow introduced into the ejector and the aerosol generating article while expanding may be effectively mixed with the aerosol generated by heating the aerosol generating article, and therefore, the aerosol flavor delivered to the user may be improved and the amount of atomization may be increased.

### Brief Description of Drawings

FIG. 1 is a perspective view of an aerosol generating device and an aerosol generating article according to an embodiment.
FIG. 2A is a front view of an ejector of the aerosol generating device shown in FIG. 1, according to an embodiment.
FIG. 2B is a front view showing a state in which the aerosol generating article is inserted into the ejector shown in FIG. 2A, according to an embodiment.
FIG. 3 is a perspective view showing another aspect of the ejector shown in FIG. 2A, according to an embodiment.
FIG. 4A is a perspective view showing another aspect of the ejector shown in FIG. 2A, according to an embodiment.
FIG. 4B is a cross-sectional view showing a state in which the ejector shown in FIG. 4A is inserted into the aerosol generating device, according to an embodiment.
FIG. 5 is a perspective view showing another aspect of the ejector shown in FIG. 2A, according to an embodiment.
FIG. 6 is a cross-sectional view of the ejector shown in FIG. 5, according to an embodiment.
FIG. 7A is a cross-sectional view schematically showing an airflow into a conventional ejector, according to an embodiment.
FIG. 7B is a cross-sectional view schematically showing an airflow into the ejector shown in FIG. 5, according to an embodiment.

### Best Mode for Carrying out the Invention

An aerosol generating device according to the present invention includes an accommodating portion in which an aerosol generating article including a cut tobacco portion is accommodated, a heater for heating the aerosol generating article accommodated in the accommodating portion, and an ejector that is detachably coupled to the accommodating portion and includes a cavity into which the aerosol generating article is inserted and at least one hole portion through which the cavity and the accommodating portion communicate with each other and the cut tobacco portion is exposed.

The at least one hole portion may include a plurality of hole portions formed on an outer circumference of the ejector, and a distance between adjacent two hole portions among the plurality of hole portions may be constant.

The at least one hole portion may have a round portion curved along a circumference of the at least one hole portion such that the aerosol generating article is prevented from escaping from the ejector through the at least one hole portion when the aerosol generating article is pushed into the cavity.

A guide portion may be formed on an outer circumferential surface of the ejector, and a groove portion that is engaged with the guide portion may be formed on the accommodating portion.

The guide portion may be engaged with the groove portion by sliding into the groove portion, and the ejector may be held in the accommodating portion by the engagement of the guide portion and the groove portion.

The guide portion may be a dot guide portion having a spherical shape.

A through hole is formed on a bottom surface of the ejector such that the bottom surface of the ejector faces one end portion of the aerosol generating article when the aerosol generating article is inserted into the cavity.

The through hole includes an expanding portion having a diameter that increases along a direction from one side of the bottom surface toward the other side of the bottom surface.

A portion of the through hole other than the expanding portion has a diameter of a constant size.

A maximum diameter of the through hole may be 0.4 times to 0.9 times a diameter of the aerosol generating article.

An aerosol generating system according to another embodiment may include an aerosol generating device according to the present invention and an aerosol generating article that is accommodated in the aerosol generating device and has a cut tobacco portion.

### Mode for the Invention

With respect to the terms in the various embodiments, the general terms which are currently and widely used are selected in consideration of functions of structural elements in the various embodiments of the present disclosure. However, meanings of the terms can be changed according to intention, a judicial precedence, the appearance of a new technology, and the like.

In addition, in certain cases, a term which is not commonly used can be selected. In such a case, the meaning of the term will be described in detail at the corresponding portion in the description of the present disclosure.

Therefore, the terms used in the various embodiments of the present disclosure should be defined based on the meanings of the terms and the descriptions provided herein.

In addition, unless explicitly described to the contrary, the word "comprise" and variations such as "comprises" or "comprising" will be understood to imply the inclusion of stated elements but not the exclusion of any other elements.

In addition, the terms "-er", "-or", and "module" described in the specification mean units for processing at least one function and operation and can be implemented by hardware components or software components and combinations thereof.

As used herein, expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, or all of a, b, and c.

It will be understood that when an element or layer is referred to as being "over," "above," "on," "connected to" or "coupled to" another element or layer, it can be directly over, above, on, connected or coupled to the other element or layer or intervening elements or layers may be present. In contrast, when an element is referred to as being "directly over," "directly above," "directly on," "directly connected to" or "directly coupled to" another element or layer, there are no intervening elements or layers present. Like numerals refer to like elements throughout.

Throughout the specification, a "longitudinal direction" of a component may be one direction in which the component extends, and the component extends longer in the one direction than in the other direction across the one direction.

Meanwhile, terms used in the present specification are for describing embodiments and are not intended to limit the embodiments. In the present specification, the singular form also includes the plural form unless otherwise specified in the phrase.

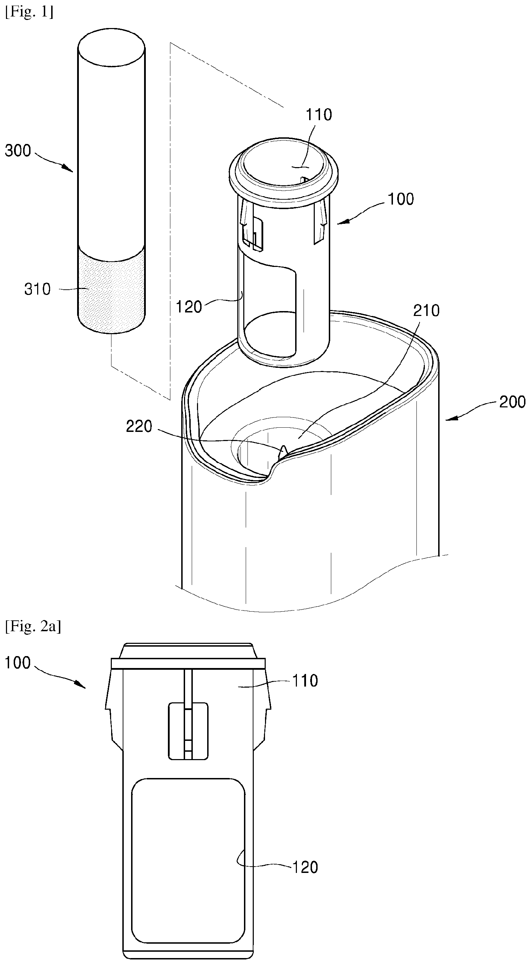

FIG. 1 is a perspective view of an aerosol generating device 200 according to an embodiment and an aerosol generating article 300.

The aerosol generating device 200 according to an embodiment includes an accommodating portion 210 in which the aerosol generating article 300 including a cut tobacco portion 310 is accommodated, a heater 220 for heating the aerosol generating article 300 accommodated in the accommodating portion 210, and an ejector that is detachably coupled to the accommodating portion 210 and includes a cavity 110 into which the aerosol generating article 300 is inserted and at least one hole portion 120 through which the cavity 110 and the accommodating portion 210 communicate with each other and the cut tobacco portion is exposed.

The aerosol generating device 200 according to an embodiment includes an accommodating portion 210 for accommodating the aerosol generating article 300 and a heater 220 for heating the aerosol generating article 300 accommodated in the accommodating portion 210.

The accommodating portion 210 may have a shape and size corresponding to the aerosol generating article 300 to accommodate the aerosol generating article 300. For example, when the aerosol generating article 300 has a cylindrical shape, the accommodating portion 210 may also have a cylindrical shaped cavity 110 to accommodate the aerosol generating article 300. However, the shapes of the aerosol generating article 300 and the accommodating portion 210 are not limited thereto, and may be changed as necessary.

The aerosol generating device 200 according to an embodiment includes a heater 220 for heating the aerosol generating article 300. The heater 220 may be arranged near the accommodating portion 210 such that teat from the heater 220 may be transferred to the accommodating portion 210 to heat the aerosol generating article 300. The aerosol generating article 300 may receive heat from the heater 220, and then an aerosol may be generated. The generated aerosol may be inhaled by a user.

The heater 220 of the aerosol generating device 200 may be, for example, an electric resistive heater 220. The heater 220 may include an electrically conductive track, and the heater 220 may be heated as current flows through the electrically conductive track. However, the heater 220 is not limited to the above described example, and may be applied without limitation as long as the heater can be heated to a desired temperature. Here, the desired temperature may be preset in the aerosol generating device 200 or set to a temperature wanted by the user.

As another example, the heater 220 may be a heater of an induction heating type. Specifically, the heater 220 may include an electrically conductive coil for heating the aerosol generating article 300 by an induction heating method, and the aerosol generating article 300 may include a susceptor capable of being heated by the heater 220 of the induction heating type.

The shape and size of the heater 220 is not limited to that shown in the drawings. The heater 220 may include at least one of a tube-type heating element, a plate-type heating element, a needle-type heating element, and a rod-type heating element and may heat an inside or an outside of the aerosol generating article 300 according to a shape of a heating element.

The aerosol generating device 200 according to an embodiment includes the ejector 100 that is detachably coupled to the accommodating portion 210 and eject the aerosol generating article 300 from the accommodating portion 210. A cavity 110 into which the aerosol generating article 300 is inserted is formed in the ejector 100.

The cavity 110 formed in the ejector 100 may have a size and shape corresponding to the size and shape of the aerosol generating article 300 inserted into the ejector 100. For example, when the aerosol generating article 300 has a cylindrical shape, the cavity 110 may have a cylindrical shape corresponding to the aerosol generating article 300 to accommodate the aerosol generating article 300 of the cylindrical shape.

The cavity 110 of the ejector 100 has a shape and size such that the aerosol generating article 300 accommodated in the ejector 100 may be removed from the aerosol generating device 200 and may contact the ejector 100. The shapes and sizes of the ejector 100 and cavity 110 are not limited to shapes and sizes shown in the drawings.

The ejector 100 is detachably coupled to the aerosol generating device 200. As an example, the ejector 100 may be detached from the aerosol generating device 200, thereby ejecting the aerosol generating article 300. As another example, the ejector 100 may eject the aerosol generating article 300 by moving a certain distance while being accommodated in the accommodating portion 210 in the aerosol generating device 200.

One end portion of the aerosol generating article 300 that is inserted into the aerosol generating device 200 according to an embodiment may be formed as a cut tobacco portion 310.

The aerosol generating article 300 may include a cut tobacco portion 310 including cut tobacco filler at one end portion. The cut tobacco filler may be tobacco containing nicotine. The cut tobacco portion 310 may be manufactured in various shapes. The cut tobacco portion 310 may be manufactured as, for example, a sheet or a strand. The cut tobacco portion 310 may be manufactured in the shape of shreds obtained by finely cutting a tobacco sheet.

The aerosol generating article 300 may include a substrate portion (not shown) including an aerosol generating material. The substrate portion may be arranged adjacent to the cut tobacco portion 310. For example, the aerosol generating material may include at least one of glycerin, propylene glycol, ethylene glycol, dipropylene glycol, diethylene glycol, triethylene glycol, tetraethylene glycol, and oleyl alcohol, but is not limited thereto.

On the outer circumferential surface of the ejector 100 of the aerosol generating device 200 according to an embodiment, at least one hole portion 120 through which the cavity 110 and the accommodating portion 210 communicate with each other is formed. The at least one hole portion 120 may be formed to extend along at least a portion of the outer circumferential surface of the ejector 100. The cavity 110 and the accommodating portion 210 communicate with each other through the at least one hole portion 120. Air, heat, etc. in the accommodating portion 210 may be introduced into the cavity 110 of the ejector 100 through the at least one hole portion 120.

Components of the aerosol generating device 200 according to an embodiment are not limited to the components illustrated in the drawings, and may further include components of the general aerosol generating device 200.

For example, the aerosol generating device 200 according to an embodiment may further include a battery (not shown) that supplies electric power to the heater 220. The aerosol generating device 200 according to an embodiment may receive electric power from the battery to heat the aerosol generating article 300.

The battery supplies electric power used for operation of the aerosol generating device 200. For example, the battery may supply electric power so that the heater 220 can be heated. In addition, the battery may supply electric power required for operating a display, a sensor, a motor, and the like, which may be installed in the aerosol generating device 200.

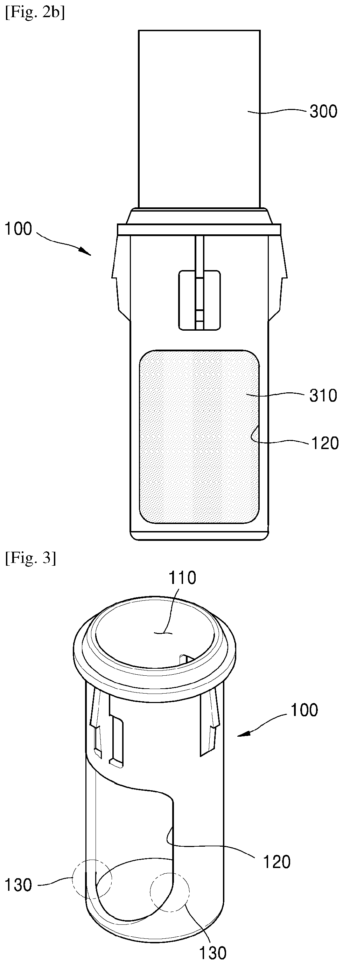

FIG. 2A is a front view of an ejector 100 of the aerosol generating device 200 shown in FIG. 1, and FIG. 2B is a front view showing a state in which the aerosol generating article 300 is inserted into the ejector 100 shown in FIG. 2A.

The hole portion 120 of the ejector 100 may be formed on a plurality of areas of the ejector 100. The plurality of hole portions 120 may be evenly spaced apart from each other. In other words, a distance between adjacent two hole portions among the plurality of hole portions may be constant. As an example, two hole portions 120 may be formed to face each other and may have the same shape.

Referring to FIG. 2B, the aerosol generating article 300 is inserted into the cavity 110 of the ejector 100.

When the aerosol generating article 300 is inserted into the cavity 110, a certain portion of the aerosol generating article 300 may be exposed to the accommodating portion 210 through the hole portion 120 at a position corresponding to the hole portion 120 of the ejector 100. At this time, the certain portion of the aerosol generating article 300 exposed through the hole portion 120 may be limited to the cut tobacco portion 310 of the aerosol generating article 300. That is, only the cut tobacco portion 310 of the aerosol generating article 300 may be exposed to the accommodating portion 210 through the hole portion 120 of the ejector 100.

The length of the cut tobacco portion 310 formed at one end portion of the aerosol generating article 300 may be longer than the length of the hole portion 120 of the ejector 100. When the aerosol generating article 300 is inserted into the cavity 110 of the ejector 100, the cut tobacco portion 310 may contact the hole portion 120.

FIG. 3 is a perspective view showing another aspect of the ejector 100 shown in FIG. 2A.

In another aspect of the ejector 100, the hole portion 120 may have a round portion 130 having a curved shape. The round portion 130 may prevent the aerosol generating article 300 from escaping from the ejector 100 through the hole portion 120 when a user inserts the aerosol generating article 300 into the cavity 110.

The round portion 130 may be curved along a portion of the circumference of the hole portion 120 in a direction closer to a center of the hole portion 120. The round portion 130 may be formed in each of the at least one hole portion 120. For example, one or more round portions 130 may be formed in a plurality of hole portions 120, respectively. The number of round portions 130 may correspond to the number of hole portions 120. The shape of the round portion 130 is not limited by the drawings and may be changed as necessary.

The round portion 130 curved along the circumference may reduce the width of the hole portion 120, and the reduced width of the hole portion 120 may be smaller than the width (e.g., diameter) of the aerosol generating article 300. Accordingly, it is possible to prevent the aerosol generating article 300 from escaping through the hole portion 120.

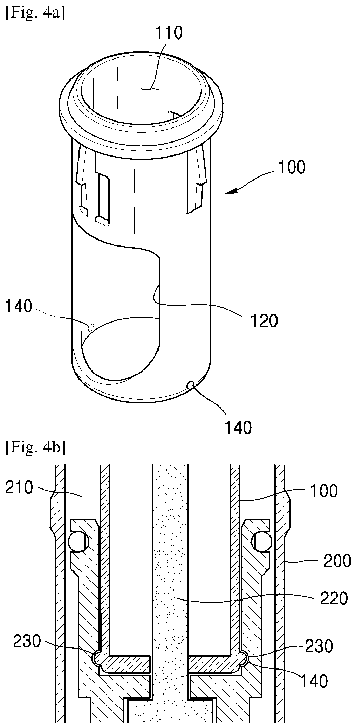

FIG. 4A is a perspective view showing another aspect of the ejector 100 shown in FIG. 2A, and FIG. 4B is a cross-sectional view showing a state in which the ejector shown in FIG. 4A is inserted into the aerosol generating device.

In another aspect of the ejector 100, at least one guide portion 140 may be formed on an outer circumferential surface of the ejector 100, and at least one groove portion 230 that is engaged with the guide portion 140 may be formed on the accommodating portion 210.

As shown in FIG. 4A, the guide portion 140 for holding the ejector 100 in the accommodating portion 210 may be formed on the outer circumferential surface of the ejector 100, and as shown in FIG. 4B, the groove portion 230 that is engaged with the guide portion 140 may be formed on the accommodating portion 210. When the ejector 100 is mounted in the accommodating portion 210, the guide portion 140 and the groove portion 230 may be engaged with each other. The ejector 100 may be held in the accommodating portion 210 by the engagement of the guide portion 140 and the groove portion 230.

When a user pushes the ejector 100 into the accommodating portion 210, the guide portion 140 may slide toward the groove portion 230 and engage with the groove portion 230.

A rail portion (not shown) in contact with the guide portion 140 may be formed on a surface of the accommodating portion 210 along the longitudinal direction of the accommodating portion 210. The guide portion 140 may be engaged with the groove portion 230 after moving along the rail portion. That is, the guide portion 140 may guide the ejector 100 so that the ejector 100 can move stably in a certain direction in the accommodating portion 210.

The guide portion 140 may be a dot guide portion 140 having a spherical shape. The guide portion 140 may have a shape of at least a portion of the sphere. A plurality of the guide portions 140 may be formed to be spaced apart from each other by a certain distance. However, the shape, number, and position of the guide portion 140 are not limited thereto, and may be changed as necessary.

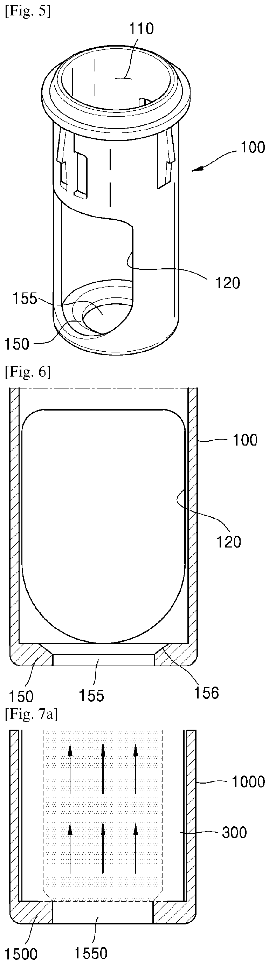

FIG. 5 is a perspective view showing another aspect of the ejector 100 shown in FIG. 2A.

As shown in FIG. 5, a through hole 155 is formed on a bottom surface 150 of the ejector 100. The bottom surface 150 of the ejector 100 is a surface facing the cut tobacco portion 310 of the aerosol generating article 300 when the aerosol generating article 300 is inserted into the cavity 110. When the aerosol generating article 300 is inserted, the bottom surface 150 of the ejector 100 may contact an end portion of the aerosol generating article 300.

When the user puffs on the aerosol generating article 300, air may flow into the aerosol generating article 300 through the through hole 155 formed on the bottom surface 150 of the ejector 100. The air flowed into the aerosol generating article 300 may then be mixed with the aerosol to be provided to the user.

FIG. 6 is a cross-sectional view of the ejector 100 shown in FIG. 5. The through hole 155 includes an expanding portion 156 that expands from one side of the bottom surface 150 toward the other side of the bottom surface150. That is, a diameter of the through hole 155 may increase along a direction from the one side of the bottom surface 150 toward the other side of the bottom surface 150 (i.e., along the longitudinal direction of the ejector 100). As shown in FIG. 6, the one side of the bottom surface 150 may be a side facing the outside of the ejector 100 based on the bottom surface 150, and the other side of the bottom surface 150 may be a side facing the inside of the ejector 100 based on the bottom surface 150.

As an example, the remaining portion of the through hole 155 other than the expanding portion 156may have a diameter of a constant size. The expanding portion 156 may be a portion closer to the inner side of the bottom surface 150.

As another example, the expanding portion 156 may be formed throughout the through hole 155. That is, the diameter of the outer circumferential surface of the through hole 155 may increase all the way from one side of the bottom surface 150 toward the other side of the bottom surface 150. The size, shape, and the like of the through hole 155 are not limited by what is shown in the drawings, and may be changed as necessary.

As the through hole 155 has the expanding portion 156 that expands along a direction from one side of the bottom surface toward the other side of the bottom surface, the through hole 155 may have a maximum diameter at a certain point. The maximum diameter of the through hole 155 may be 0.4 times to 0.9 times the diameter of the aerosol generating article 300.

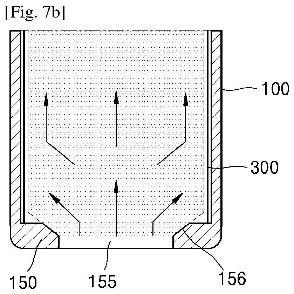

FIG. 7A is a cross-sectional view schematically showing an airflow into a conventional ejector 1000, and FIG. 7B is a cross-sectional view schematically showing an airflow into the ejector 100 shown in FIG. 5.

Referring to FIG. 7A showing the conventional ejector 1000 and the airflow into the conventional ejector 1000, a through hole 1550 may be formed on a bottom surface 1500 of the conventional ejector 1000. At this time, a diameter of the through hole 1550 formed on the bottom surface 1500 of the conventional ejector 1000 may have a constant size. That is, in the conventional ejector 1000, the through hole 1550 may be formed to have a cylindrical shape of the same diameter.

Airflow which has passed through the through hole 1550 having the same diameter may not uniformly flow into the aerosol generating article 300. Accordingly, it is difficult for the airflow to be effectively mixed with an aerosol generated by heating the aerosol generating article 300, and thus the flavor of the aerosol delivered to the user may be reduced and the amount of atomization may be reduced.

FIG. 7B shows the ejector 100 of the aerosol generating device 200 and the airflow into the ejector 100 according to an embodiments. As shown in FIG. 7B, the through hole 155 is formed on the bottom surface 150 of the ejector 100 of the aerosol generating device 200, and the through hole 155 has the expanding portion 156 that expands along a direction from one side of the bottom surface 150 of the ejector 100 toward the other side of the bottom surface 150.

The airflow that passed through the through hole 155 having the expanding portion 156 may expand inside the ejector 100 along the inclined wall of the through hole 155 which corresponds to the expanding portion 156. Accordingly, pressure loss in the ejector 100 and in the aerosol generating article 300 is prevented, and an airflow path is expanded to increase the inflow of air.

The airflow introduced into the ejector 100 and the aerosol generating article 300 while expanding may be effectively mixed with an aerosol generated by heating the aerosol generating article 300, and therefore, the flavor of the aerosol delivered to the user may be improved and the amount of atomization may be increased.

An aerosol generating system according to an embodiment may include an aerosol generating device 200 and an aerosol generating article 300 that is accommodated in the aerosol generating device 200 and has a cut tobacco portion 300. At this time, the configurations and effects of the aerosol generating device 200 and the aerosol generating article 300 are the same as described above, and therefore, a detailed description in the overlapping range will be omitted.

The aerosol generating device 200 according to the present embodiments include an ejector 100 having at least one hole portion 120 formed on an outer circumferential surface. The inside (i.e., the cavity 110) and the outside (i.e., the accommodating portion 210) of the ejector 100 communicate with each other through the at least one hole portion 120 of the ejector 100. Heat generated outside the ejector 100 may be efficiently transferred into the ejector 100 through the at least one hole portion 120. Efficient heat transfer into the ejector 100 may enhance the flavor of the generated aerosol, increase the amount of atomization, and eliminate the imbalance of heating the aerosol generating article 300.

In the aerosol generating device 200 according to the embodiments, it may be easy to access to the inside of the ejector 100 through the at least one hole portion 120. Through the access inside the ejector 100, the user may easily remove the residues deposited in the ejector 100. As such, the user can maintain the performance of the aerosol generating device 200 and prevent the aerosol generating device 200 from being damaged due to the residues. In addition, it is possible to prevent health and hygiene problems that may occur due to the residues deposited on the aerosol generating device 200.

The disclosed methods should be considered in descriptive sense only and not for purposes of limitation.

## Claims

1. An aerosol generating device (200) comprising:
an accommodating portion (210) configured to accommodate an aerosol generating article (300) including a cut tobacco portion (310);
a heater (220) configured to heat the aerosol generating article (300) accommodated in the accommodating portion (210); and
an ejector (100) that is detachably coupled to the accommodating portion (210), and includes:
a cavity (110) into which the aerosol generating article (300) is insertable; and
a through hole (155),
wherein the through hole (155) is formed in a bottom surface (150) of the ejector (100) and the bottom surface (150) faces one end portion of the aerosol generating article (300) when the aerosol generating article (300) is inserted into the cavity (110), and
wherein the through hole (155) includes an expanding portion (156) having a diameter that increases along a direction from one side of the bottom surface (150) toward the other side of the bottom surface (150),
***characterized in that***
the ejector (100) comprises at least one hole portion (120) through which the cavity (110) and the accommodating portion (210) communicate and the cut tobacco portion can be exposed.

2. The aerosol generating device of claim 1, wherein
the at least one hole portion (120) includes a plurality of hole portions, and
a distance between adjacent two hole portions (120) among the plurality of hole portions is constant.

3. The aerosol generating device of claim 1, wherein the at least one hole portion (120) has a round portion (130) curved along a circumference of the at least one hole portion (120) such that the aerosol generating article (300) is prevented from escaping from the ejector (100) through the at least one hole portion (120) when the aerosol generating article (300) is pushed into the cavity (110).

4. The aerosol generating device of claim 1, wherein
a guide portion (140) is formed on an outer circumferential surface of the ejector (100), and
a groove portion (230) configured to engage with the guide portion (140) is formed on the accommodating portion (210).

5. The aerosol generating device of claim 4, wherein
the guide portion (140) is configured to engage with the groove portion (230) by sliding into the groove portion (230), and
the ejector (100) is held in the accommodating portion (210) by engagement of the guide portion (140) and the groove portion (230).

6. The aerosol generating device of claim 4, wherein the guide portion (140) is a dot guide portion having a spherical shape.

7. The aerosol generating device of claim 1, wherein a portion of the through hole other than the expanding portion has a diameter of a constant size.

8. The aerosol generating device of claim 1, wherein a maximum diameter of the through hole (155) is 0.4 times to 0.9 times a diameter of the aerosol generating article (300).

9. An aerosol generating system comprising:
the aerosol generating device (200) according to any one of claims 1 to 8; and
the aerosol generating article (300) configured to be accommodated in the aerosol generating device (200) and has the cut tobacco portion (310).

## Patentansprüche

1. Aerosolerzeugungsvorrichtung (200), die umfasst:
einen Aufnahmeabschnitt (210), der konfiguriert ist, einen aerosolerzeugenden Artikel (300) aufzunehmen, der einen ausgeschnittenen Tabakabschnitt (310) enthält;
ein Heizgerät (220), das konfiguriert ist, den aerosolerzeugenden Artikel (300), der in dem Aufnahmeabschnitt (210) untergebracht ist, zu erwärmen; und
eine Ausstoßeinrichtung (100), die abnehmbar an den Aufnahmeabschnitt (210) gekoppelt ist und enthält:
einen Hohlraum (110), in den der aerosolerzeugende Artikel (300) einführbar ist; und
ein Durchgangsloch (155),
wobei das Durchgangsloch (155) in einer unteren Fläche (150) der Ausstoßeinrichtung (100) gebildet ist und die untere Fläche (150) einem Endabschnitt des aerosolerzeugenden Artikels (300) zugewandt ist, wenn der aerosolerzeugende Artikel (300) in den Hohlraum eingeführt ist, und
wobei das Durchgangsloch (155) einen sich ausdehnenden Abschnitt (156) mit einem Durchmesser, der entlang einer Richtung von einer Seite der unteren Fläche (150) in Richtung der anderen Seite der unteren Fläche (150) zunimmt, enthält,
**dadurch gekennzeichnet, dass**
die Ausstoßeinrichtung (100) mindestens einen Lochabschnitt (120) umfasst, durch den der Hohlraum (110) und der Aufnahmeabschnitt (210) kommunizieren und der ausgeschnittene Tabakabschnitt freigelegt werden kann.

2. Aerosolerzeugungsvorrichtung nach Anspruch 1, wobei
der mindestens eine Lochabschnitt (120) mehrere Lochabschnitte umfasst und
ein Abstand zwischen benachbarten zwei Lochabschnitten (120) unter den mehreren Lochabschnitten konstant ist.

3. Aerosolerzeugungsvorrichtung nach Anspruch 1, wobei der mindestens eine Lochabschnitt (120) einen runden Abschnitt (130) besitzt, der entlang eines Umfangs des mindestens einen Lochabschnitts (120) derart gekrümmt ist, dass verhindert wird, dass der aerosolerzeugende Artikel (300) von der Ausstoßeinrichtung (100) durch den mindestens einen Lochabschnitt (120) entweicht, wenn der aerosolerzeugende Artikel (300) in den Hohlraum (110) geschoben wird.

4. Aerosolerzeugungsvorrichtung nach Anspruch 1, wobei
ein Führungsabschnitt (140) auf einer äußeren Umfangsfläche der Ausstoßeinrichtung (100) gebildet ist und
ein Rillenabschnitt (230), der konfiguriert ist, mit dem Führungsabschnitt (140) in Eingriff zu gelangen, auf dem Aufnahmeabschnitt (210) gebildet ist.

5. Aerosolerzeugungsvorrichtung nach Anspruch 4, wobei
der Führungsabschnitt (140) konfiguriert ist, mit dem Rillenabschnitt (230) durch Gleiten in den Rillenabschnitt (230) in Eingriff zu gelangen, und
die Ausstoßeinrichtung (100) in dem Aufnahmeabschnitt (210) durch einen Eingriff des Führungsabschnitts (140) und des Rillenabschnitts (230) gehalten wird.

6. Aerosolerzeugungsvorrichtung nach Anspruch 4, wobei der Führungsabschnitt (140) ein Punktführungsabschnitt mit einer Kugelform ist.

7. Aerosolerzeugungsvorrichtung nach Anspruch 1, wobei ein Abschnitt des Durchgangslochs, der von dem sich ausdehnenden Abschnitt verschieden ist, einen Durchmesser einer konstanten Größe besitzt.

8. Aerosolerzeugungsvorrichtung nach Anspruch 1, wobei ein maximaler Durchmesser des Durchgangslochs (155) gleich dem 0,4- bis 0,9-fachen Durchmesser des aerosolerzeugenden Artikels (300) ist.

9. Aerosolerzeugungssystem, das umfasst:
die Aerosolerzeugungsvorrichtung (200) nach einem der Ansprüche 1 bis 8; und
den aerosolerzeugenden Artikel (300), der konfiguriert ist, in der Aerosolerzeugungsvorrichtung (200) untergebracht zu werden, und der den ausgeschnittenen Tabakabschnitt (310) aufweist.

## Revendications

1. Dispositif de production d'aérosol (200) comportant :
une partie de réception (210) configurée pour recevoir un article de production d'aérosol (300) incluant une partie de tabac haché (310) ;
un élément chauffant (220) configuré pour chauffer l'article de production d'aérosol (300) reçu dans la partie de réception (210) ; et
un éjecteur (100) qui est couplé à la partie de réception (210) de manière détachable, et inclut :
une cavité (110) dans laquelle l'article de production d'aérosol (300) peut être inséré ; et
un trou traversant (155),
dans lequel le trou traversant (155) est formé sur une surface de fond (150) de l'éjecteur (100) et la surface de fond (150) est dirigée vers une partie d'extrémité de l'article de production d'aérosol (300) lorsque l'article de production d'aérosol (300) est inséré dans la cavité (110), et
dans lequel le trou traversant (155) inclut une partie d'élargissement (156) ayant un diamètre qui augmente le long d'une direction allant d'un côté de la surface de fond (150) vers l'autre côté de la surface de fond (150),
**caractérisé en ce que**
l'éjecteur (100) comporte au moins une partie de trou (120) par laquelle la cavité (110) et la partie de réception (210) communiquent et la partie de tabac haché (310) peut être exposée.

2. Dispositif de production d'aérosol selon la revendication 1, dans lequel
la au moins une partie de trou (120) inclut une pluralité de parties de trou, et
une distance entre deux parties de trou (120) adjacentes parmi la pluralité de parties de trou est constante.

3. Dispositif de production d'aérosol selon la revendication 1, dans lequel la au moins une partie de trou (120) a une partie ronde (130) incurvée le long d'une circonférence de la au moins une partie de trou (120) de telle sorte que l'article de production d'aérosol (300) est empêché de s'échapper de l'éjecteur (100) par la au moins une partie de trou (120) lorsque l'article de production d'aérosol (300) est poussé dans la cavité (110).

4. Dispositif de production d'aérosol selon la revendication 1, dans lequel
une partie de guidage (140) est formée sur une surface circonférentielle extérieure de l'éjecter (100), et
une partie de rainure (230) configurée pour engager la partie de guidage (140) est formée sur la partie de réception (210).

5. Dispositif de production d'aérosol selon la revendication 4, dans lequel
la partie de guidage (140) est configurée pour s'engager dans la partie de rainure (230) en glissant dans la partie de rainure (230), et
l'éjecteur (100) est maintenu dans la partie de réception (210) par l'engagement de la partie de guidage (140) et de la partie de rainure (230).

6. Dispositif de production d'aérosol selon la revendication 4, dans lequel la partie de guidage (140) est une partie de guide punctiforme ayant une forme sphérique.

7. Dispositif de production d'aérosol selon la revendication 1, dans lequel une partie du trou traversant autre que la partie d'élargissement, a un diamètre d'une taille constante.

8. Dispositif de production d'aérosol selon la revendication 1, dans lequel un diamètre maximal du trou traversant (155) est de 0,4 fois à 0,9 fois un diamètre de l'article de production d'aérosol (300).

9. Système de production d'aérosol comportant :
le dispositif de production d'aérosol (200) selon l'une quelconque des revendications 1 à 8 ; et
l'article de production d'aérosol (300) configuré pour être reçu dans le dispositif de production d'aérosol (200) et ayant la partie de tabac haché (310).
